(19) Europäisches Patentamt
European Patent Office
Office européen des brevets

(11) **EP 2 618 307 B1**

(12) **EUROPEAN PATENT SPECIFICATION**

(45) Date of publication and mention
of the grant of the patent:
**04.03.2015 Bulletin 2015/10**

(51) Int Cl.:
**G06T 7/00** (2006.01)   **G06T 7/60** (2006.01)

(21) Application number: **13151645.2**

(22) Date of filing: **17.01.2013**

(54) **Method and system for determining the volume of epicardial fat from volumetric images, and corresponding computer program**

Verfahren und System zur Bestimmung des Volumens von epikardialem Fett aus volumetrischen Bildern sowie zugehöriges Computerprogramm

Procédé et système permettant de déterminer le volume de matière grasse épicardique à partir d'images volumétriques et programme informatique correspondant

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2012 IT TO20120030**

(43) Date of publication of application:
**24.07.2013 Bulletin 2013/30**

(73) Proprietor: **Consiglio Nazionale Delle Ricerche 00185 Roma (IT)**

(72) Inventors:
 • **Coppini, Giuseppe**
  **I-56011 Calci (Pisa) (IT)**
 • **Favilla, Riccardo**
  **I-56019 Vecchiano (Pisa) (IT)**
 • **Marraccini, Paolo**
  **I-56121 Pisa (IT)**
 • **Salvetti, Ovidio**
  **I-56017 Ghezzano, San Giuliano Terme (Pisa) (IT)**
 • **Moroni, Davide**
  **I-56011 Calci (Pisa) (IT)**
 • **Pieri, Gabriele**
  **I-56011 Calci (Pisa) (IT)**

(74) Representative: **Deambrogi, Edgardo et al
Corso Emilia 8
10152 Torino (IT)**

(56) References cited:

• **Guiseppe Coppini ET AL: "P2505 - Computer technology for the quantification of pericardial fat assessed through cardiac CT", ESC Congress 2010, Stockholm, Sweden, 28 August 2010 (2010-08-28), pages 436-436, XP055027988, Sophia Antipolis, France DOI: 10.1093/eurheartj/ehq288 Retrieved from the Internet: URL:http://spo.escardio.org/eSlides/ view.a spx?eevtid=40&fp=P2505 [retrieved on 2012-05-23]**
• **GUISEPPE COPPINI ET AL: "Computational methods for pericardial fat evaluation", MDA 2011 - ADVANCES IN MASS DATA ANALYSIS OF IMAGES AND SIGNALS IN MEDICINE, BIOTECHNOLOGY, CHEMISTRY AND FOOD INDUSTRY. 6TH INTERNATIONAL CONFERENCE, 1 January 2011 (2011-01-01), pages 38-49, XP055028865, Fockendorf, Germany ISBN: 978-3-94-295202-6**
• **COPPINI G ET AL: "Regional Epicardial Fat Measurement: Computational Methods for Cardiac CT Imaging", SELECTED PAPERS FROM THE INTERNATIONAL CONFERENCE ON MASS DATA ANALYSIS OF SIGNALS AND IMAGES IN MEDICINE, BIOTECHNOLOGY, CHEMISTRY, AND FOOD INDUSTRY, MDA; [TRANSACTIONS ON MASS-DATA ANALYSIS OF IMAGES AND SIGNALS], LEIPZIG : INST. OF COMPUTER VISIO, vol. 1, no. 1, 1 September 2009 (2009-09-01), pages 101-110, XP009159668, ISBN: 978-3-940501-06-6**

- **GIUSEPPE COPPINI: "Quantification of Epicardial Fat by Cardiac CT Imaging", THE OPEN MEDICAL INFORMATICS JOURNAL, vol. 4, no. 1, 27 July 2010 (2010-07-27), pages 126-135, XP055027971, ISSN: 1874-4311, DOI: 10.2174/1874431101004010126**

**Description**

The present invention relates to quantitative clinical diagnostic methods and systems, particularly for image processing, and specifically for the determination of real dimensional parameters of anatomical structures from volumetric images such as tomographic or magnetic resonance images, which can differentiate fatty tissues from other tissue components.

More specifically, it relates to a method for determining the volume of epicardial fat from volumetric images according to the preamble of Claim 1, and to a processing system and a computer program for implementing the aforesaid method.

Pericardial fat, like other visceral fat locations, is correlated with important cardiovascular and metabolic pathologies and is considered to be a significant indicator of the corresponding risk factors. Numerous clinical studies have demonstrated the importance of the measurement of epicardial fat as an independent prognostic risk factor. The accumulation of visceral fat, and particularly epicardial fat, is considered to be a condition which promotes the inappropriate production of adipose endocrine factors such as adipokines which may affect tissue remodelling, the activation of inflammatory processes, and cardiac function. The quantification of epicardial fat can provide an independent objective parameter which is useful for prognostic stratification and longitudinal cardiovascular risk evaluation.

However, there are as yet no known methods for making a reliable and reproducible measurement of the volume of cardiac fat which are universally accepted and simple to use.

To this end, numerous studies have been conducted, using echocardiographic image acquisition, magnetic resonance imaging and tomographic imaging (cardiac CT).

Cardiac CT can be used to acquire images of the whole volume of the heart over the whole cardiac cycle, with good spatial resolution and excellent density resolution. The coronary vessels can thus be visualized by non-selective angiography procedures, and this has promoted the widespread use of cardiac CT methods. Numerous clinical studies have been conducted using computer tomography to evaluate the extent of fatty deposits. In these studies, the evaluation of fat was typically conducted by manually segmenting the two-dimensional images. However, this approach is subject to numerous sources of variability and is not suitable for routine use.

Other studies are limited to the simple evaluation of the thickness of the fat layer, which does not always provide an adequate and objective measurement of the volume of cardiac fat.

There are also known methods of estimating the volume of cardiac fat based on image processing methods. For example Bandekar, A.N., Naghavi, M., and Kakadiaris, I.A., in "Automated Pericardial Fat Quantification in CT Data," Engineering in Medicine and Biology Society, 2006. EMBS '06. 28th Annual International Conference of the IEEE , pp. 932-935, (2006), describe an automatic method based on textural features, which provides an estimate of the total mediastinal fat. Similar results were obtained by Damini Dey, Yasuyuki Suzuki, Shoji Suzuki, Muneo Ohba, Piotr J. Slomka, Donna Polk, Leslee J. Shaw, and Daniel S. Berman, in "Automated Quantization of Pericardiac Fat From Noncontrast CT", Investigative Radiology Volume 43, number 2, February 2008, using an approach based on multi-threshold region growing methods. Additionally, Coppini G, Favilla R, Lami E, Marraccini P, Moroni D, and Salvetti O., in "Regional epicardial fat measurement: Computational methods for cardiac CT imaging", Transactions on Mass-Data Analysis of Images and Signals, Vol. 1:101-110 (2009), and Coppini G, Favilla R, Marraccini P., Moroni D., and Pieri G., in "Quantification of Epicardial Fat by Cardiac CT Imaging", The Open Medical Informatics Journal, vol. 4 pp. 126 - 135 (2010), describe methods for manual segmentation of the epicardial surface, where the epicardial fat is then extracted automatically using a model based on level sets theory. It should be noted that the last two studies are based on manual slice-by-slice extraction of the contours of the heart, and the approach followed is strictly two-dimensional. In particular, the 2D images corresponding to each slice are processed individually in order to calculate the area corresponding to fat, while the total volume is calculated by integrating the values of the areas.

A method for determining the volume of epicardial fat of a heart according to the preamble of claim 1 is disclosed by G. Coppini at al. in "P2505 - Computer technology for the quantification of pericardial fat assessed though cardiac CT", ESC Congress 2010, Stockholm, Sweden, and by G. Coppini et al. in "Computational methods for pericardial fat evaluation", MDA 2011 - Advances in Mass data Analyses of Images and Signals in Medicine, Biotechnology, Chemistry and food Industry, 6th Internatinal Conference, 1 January 2011, pages 38-49.

The object of the present invention is therefore to provide an easily used method for obtaining a reliable and reproducible measurement of the volume of cardiac fat, based on volumetric images, such as tomographic images, or on a plurality of available standard scans obtained by computer tomography of the heart, acquired with or without the use of contrast medium, for example in the course of coronary CT angio examinations, the method being usable in clinical practice.

According to the present invention, this object is achieved by means of a method for determining the volume of epicardial fat having the characteristics claimed in Claim 1.

Specific embodiments are described in the dependent claims, the content of which is to be considered as an integral part of the present description.

The invention also proposes a system for determining the volume of epicardial fat, a corresponding computer program, and a computer program product, as claimed.

Briefly, the present invention is based on the principle of estimating the epicardial surface by means of a three-dimensional model of series expansion of vector spherical harmonic functions from a series of volumetric images, such as tomographic scans comprising the cardiac volume, acquired with or without contrast medium, and calculating the cardiac fat volume on the basis of the volume elements or voxels which are located within the epicardial surface estimated in this way and which have an attenuation level or grey levels within a predetermined range which is characteristic of fatty tissue.

Starting from the collection of images, the estimation of the epicardial surface may be guided by the preliminary identification, which may be manual or automatic, of a plurality of reference contours of the heart, representative of the heart as a whole, for example the contour of the heart which can be derived from a pair of views taken on a long axis (four chambers and two chambers), and from at least a pair of views taken on a short axis, extracted in the basal and apical regions.

The coefficients of the series expansion of vector spherical harmonic functions may be calculated by minimizing a predetermined cost functional.

Further characteristics and advantages of the invention will be disclosed more fully in the following detailed description of one embodiment of the invention for X-ray (CT) images, provided by way of non-limiting example, with reference to the attached drawings, in which:

Figure 1 is a schematic representation of a processing system programmed for the implementation of the method according to the invention;

Figure 2 is a flow diagram of the method proposed by the invention;

Figure 3 shows a diagram of the heart with the positions of the various planes of interest and examples of images of the corresponding tomographic cross sections;

Figure 4 shows a series of tomographic images representing differently orientated views of the heart, on which the reference contours of the heart are shown;

Figure 5 is a diagram representing the distribution of the grey levels of the voxels within the estimated surface of the heart;

Figure 6 shows two three-dimensional views (a left-hand front view and a righthand rear view) of the epicardial fat reconstructed from tomographic images, processed by the method proposed by the invention;

Figure 7 shows, from the top left to the top right, the two-chamber and four-chamber views with corresponding lines of the atrioventricular plane, and, at the bottom, two views on a short axis with the lines of the interventricular plane; and

Figure 8 shows (i) a plate with four three-dimensional views of the epicardial fat in the ventricular region, and (ii) views of the right and left cross sections of the ventricular fat deposits, according to the described method.

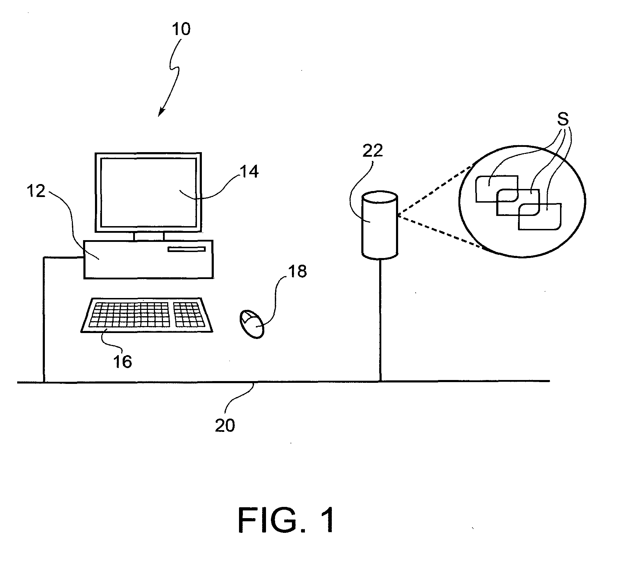

With reference to Figure 1, a system for determining the volume of epicardial fat from volumetric images, including but not limited to tomographic images, is shown in its essential outlines. It may comprise, for example, a computer workstation 10 of a known type, having a processor subsystem (base module) 12, a display device 14, a keyboard 16, a pointing device (mouse) 18 and a device for connection to a local network (network bus) 20.

An example of a workstation 10 which can be used is the Mac Mini model, made by Apple, Inc., which has an Intel Core 2 Duo 2.0 GHz CPU, 4 GB RAM, a 250 GB hard disc and a MacOsX 10.6 operating system.

The workstation may be arranged to run a program or groups of programs stored on the hard disc or accessible via the network, and to show the results on the display 14. The program or groups of programs may be processing and calculation programs which implement the method according to the invention, as will be described in detail below.

The system according to the invention may further comprise a storage memory subsystem 22, of a known type, integrated with the workstation 10 or connected thereto by means of the network connection 20, and adapted to store databases of tomographic images, as described in detail below, with reference to the implementation of the method according to the invention.

Evidently, the databases can also be stored, if they are of limited size, in the hard disc of the workstation 10 without any modification of the characteristics of the invention. The system may also be arranged for connection to other peripheral input/output devices, local or remote, or may include other computer system configurations, such as a multiprocessor system or a computer system of the distributed type, where the tasks may be executed by remote computer devices interconnected by a communications network and the modules of the program can be stored in both local and remote storage devices.

The invention further proposes a computer program or group of programs, in particular a computer program on, or in, a data medium or memory, adapted to implement the invention. This program may use any programming language, and may be in the form of source code, object code or a code intermediate between source and object code, for example in a partially compiled form, or in any other desired form for implementing the method according to the

**EP 2 618 307 B1**

invention.

Finally, the embodiments of the invention further comprise a computer program product, which may be a computer storage medium which is readable by a computer system and which encodes a program or group of programs of computer instructions for executing the processing of data representing volumetric images. Specific non-limitative examples of a computer-readable data medium are any object or device capable of storing a program, such as a random access memory, a read-only memory, a compact disc memory, or a magnetic recording medium or a hard disc. More generally, the computer program product may also be in the form of a data stream readable by a computer system, which encodes a computer instructions program, and which can be carried, for example, on a network, such as the Internet.

The solutions disclosed here are considered to be well-known in the art and will not be described further.

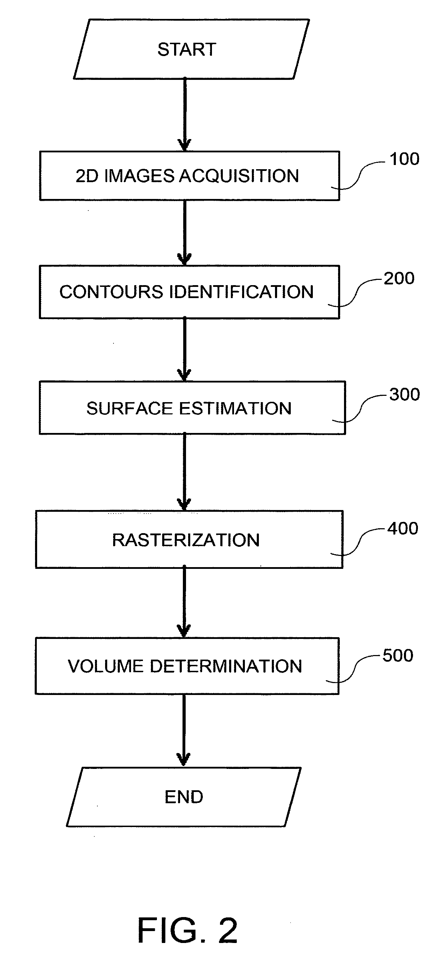

With reference to the flow diagram of Figure 2, the method according to the invention includes initially, in step 100, the acquisition of a collection of two-dimensional image data, such as computer tomography image data S, representing a plurality of views, for example successive plane views (or slices) of the heart, typically also including images of adjacent organs or tissues. The collection of image data is, for example, obtained at a time other than the time of implementation of this method, and, as mentioned above, is stored in the storage memory subsystem 22.

In general, a complete cardiac CT scan is defined by a series of two-dimensional images acquired orthogonally to the axis of the scanning device (axis z). Each of these images represents the attenuation of the scanning X-rays in a tissue slice of predetermined thickness (in the present case, typical thicknesses range from about 2.5 mm to about 0.5 mm). Considered as a whole, stacked along the axis z, the slices define a function I(x,y,z) in the reference system of the scanning device in which I is the value of the attenuation, typically expressed in Hounsfield units (HU), at the point with coordinates x,y,z. The individual images (slices) generally correspond to I(x,y,z) for fixed values of z.

When the tomographic images have been acquired, they are initially converted into a suitable format for processing by an image representation and displaying program.

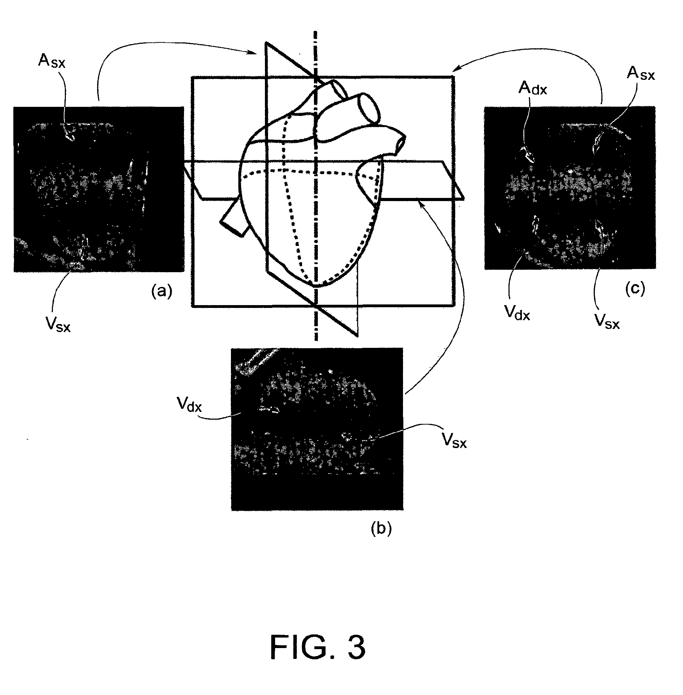

Starting with images S selected from the available series, including at least the whole cardiac volume, an operation (200) of morphological characterization of the volume of interest is carried out, including the identification of at least three two-dimensional contours P1-P3 (and more generally a number n of profiles P1-PN) of the heart, this identification being performed, respectively, in at least one sectional image taken along a short axis at the base of the ventricle and in two sectional images taken along a long axis, (representing four chambers and two chambers respectively) of the heart. Figure 3 shows a representation of a heart and the cross-sectional planes of interest. In Figure 3, the images (a), (b) and (c) show examples of CT images which are, respectively, (a) a sectional image taken on the long axis, showing the two left chambers, namely the left atrium Asx and the left ventricle Vsx, (b) a sectional image taken on the short axis, showing the two lower chambers, namely the right ventricle Vdx and the left ventricle Vsx, and (c) a sectional image taken on the long axis, showing all four chambers, namely the right atrium Adx, the left atrium Asx, the right ventricle Vdx, and the left ventricle Vsx.

Because of the regularity and symmetry, even if only approximate, of the epicardial surface, the use of other contours from long-axis images does not appear to be justified. As regards the use of other contours from short-axis cross sections, it has been found experimentally that the addition of another contour in the medio-apical position may improve the estimate in individual cases. However, experimental observations suggest that there is no benefit in using more than three contours obtained in a short axis. It should also be borne in mind that an increase in the number of contours (and therefore of points used) generally leads to an increase in calculation time.

The number of contours to be traced therefore typically varies from a minimum of 3 (2 long-axis and 1 short-axis), sufficient for hearts with a regular anatomy, to a maximum of 5 (2 long-axis and 3 short-axis). The use of two long-axis sections and two short-axis sections appears to be sufficient for general use.

In view of the above considerations, in a currently preferred embodiment, the selected sections are at least one short-axis section taken in a basal region (optionally a short-axis section in the medio-apical region is used), a long-axis section corresponding to the four-chamber view, and a long-axis section corresponding to the two-chamber view, corresponding to the left ventricle and atrium.

The contours can be identified automatically, for example by implementing an automatic shape recognition method such as a method for automatic segmentation of the cardiac volume, from the converted image data, and executed on the basis of algorithms described in the literature, or in assisted (semi-automatic) mode, or again by means of a manual operation performed by an operator for plotting a contour curve marking a discrete number of boundary points. In assisted or automatic mode, the segmentation method makes use of the image gradient to locate the contours, subject to the limitations of current methods which allow reliable recognition only in respect of certain regions of the contour (such as the boundary between the left ventricle and the lung).

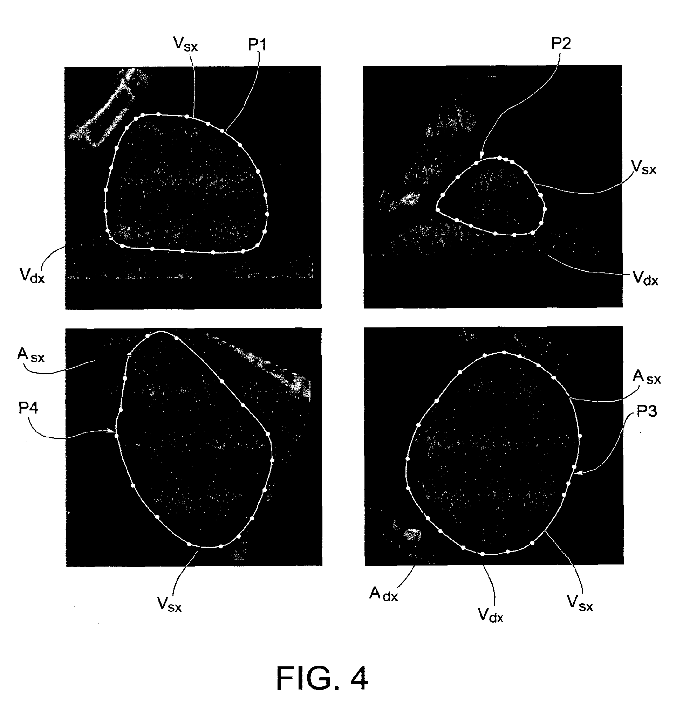

Figure 4 shows, in clockwise order starting from the upper left, four images comprising the volume of interest, namely a first short-axis section of the basal region of the left ventricle Vsx and the right ventricle Vdx, a second short-axis section of the apical region of the same left ventricle Vsx and right ventricle Vdx, a first long-axis section including the view of all four chambers Asx, Adx, Vsx, Vdx, and a second long-axis section including the view of two chambers, namely the left atrium Asx and the left ventricle Vsx.

The contour curves P1-P4, which interpolate a predetermined number of discrete boundary points by means of cubic splines, are plotted on the images of Figure 4. As a general rule, about 20 boundary points may be required to identify the long-axis contours, about 15 boundary points are required for a short-axis section in a basal region, and about 10 boundary points are required for a short-axis section in an apical region.

D = {Di, i=1, ..., K} denotes the set of all the points of the contours plotted in the common reference system.

In the next step 300, a 3D estimate is made of the epicardial surface of the whole pericardium, from the points in D of the plotted contours. For this purpose, the centroid G of the points in D is calculated, and the origin of the reference system is translated to G. The spherical coordinates relative to the pole G are also indicated by $(\varphi, \theta)$. In the following text, the symbol $\Sigma$ denotes the epicardial surface and $\Omega$ denotes the domain enclosed by it. As a general rule, $\Sigma$ can be expressed in parametric form as $x(\varphi, \theta)$, and is represented here by a series of spherical harmonics.

$$\mathbf{x}(\varphi, \theta) = \sum_{n=0}^{N} \sum_{m=0}^{n} \left[ \mathbf{a}_{mn} \cos m\varphi + \mathbf{b}_{mn} \sin m\varphi \right] P_{mn}(\cos(\theta))$$

where $\mathbf{a}_{mn}$, $\mathbf{b}_{mn}$ are the (vector) coefficients of the series and $P_{mn}(t)$ are the associated Legendre polynomials of degree n and order m. The number N is the order of the surface. In the present case, in view of the (approximate) regularity and symmetry of the epicardium, a low order N is expected to be suitable: experimental evidence indicates that a value of N of 2-3 is typically suitable for this purpose. However, for the procedure of estimating the coefficients of the series of spherical harmonics as described below, it is not necessary to specify the "exact" value of the order in advance. Nevertheless it should be borne in mind that implementation will be more efficient when the order of the surface is limited than when the order is too high, and this will also advantageously limit the calculation time. For this reason, N = 3 was used in the embodiment.

The aim is find a regular surface which approximates the points in D. It is also assumed that the voxels within the surface can represent blood (or contrast medium if appropriate), cardiac muscle and epicardial fat: it is possible to identify with certainty a value $I_{high}$ of the grey levels which is acceptable as belonging to $\Omega$. Similarly, it is possible to identify a value $I_{low}$ below which the voxels cannot belong to $\Omega$. Another piece of information which can be used relates to the regions where there are high grey level gradients (the heart-lung interface) which belong in almost all cases to the epicardial surface.

On the basis of these considerations, the coefficients of the series are estimated by numerically minimizing preferably the following cost functional:

$$\mathcal{C}(a_{mn}, b_{mn}) = \alpha \mathcal{P}(a_{mn}, b_{mn}) + \beta \mathcal{G}(a_{mn}, b_{mn}) + \gamma \mathcal{E}(a_{mn}, b_{mn}) + \delta \mathcal{S}(a_{mn}, b_{mn})$$

in which the coefficients $\alpha$, $\beta$, $\gamma$, $\delta$ may be selected as explained below, while the terms $\mathcal{P}$, G', $\varepsilon$, S are defined as follows:

- $\mathcal{P}$ is the approximation error, calculated as the sum of the distances from the surface $\Sigma$ of the points $P_i$, according to the following expression:

$$\mathcal{P} = \frac{1}{K} \sum_i d_i^2(D_i, \Sigma)$$

where K is the number of points $D_i$
- G' penalizes the presence within the surface of "unexpected" grey levels, according to the expression

$$\mathcal{G} = \int_\Omega H(I_{high} - I(x,y,z))(I_{high} - I(x,y,z))^2 dxdydz +$$

$$+ \int_\Omega H(I(x,y,z) - I_{low})(I_{low} - I(x,y,z))^2 dxdydz$$

where H is the unitary step function and $I_{low}$ and $I_{high}$ represent, respectively, the lower and upper bounds of the grey levels of the fat window (for example, in the case of CT, $I_{low} \approx$ - 200 HU and $I_{high} \approx$ 40 HU). It should be noted that the first function to be integrated is cancelled for I(x,y,z) > $I_{high}$, while the second is cancelled for I(x,y,z) < $I_{low}$.

- $\varepsilon$ indicates the coincidence of the surface with the regions having high grey gradients (heart/lung interface), according to the expression:

$$\mathcal{E} = \int_\Sigma \frac{1}{1 + |\nabla I(x,y,z)|^2} |\nabla I(x,y,z)| d\Sigma$$

the integral being calculated on the surface $\Sigma$. In the low-gradient regions it has a considerable weight, while in the edges the contribution to the integral is lower. It appears to be convenient to use the Gaussian gradient for the calculation of the gradient.

- $S$ ensures the regularity of the surface, by making the coefficients of the series to decrease rapidly as the order increases, according to the expression:

$$\mathcal{S} = \sum_n \sum_m w_{nm}(a^2_{mn} + b^2_{mn})$$

In particular, it is assumed that $w_{mn} = k \exp(n/N)$, resulting in an exponential decrease in the coefficients of the series.

The coefficients $\alpha$, $\beta$, $\gamma$, $\delta$ may be selected on the understanding that the constraints of approximation and regularity are stronger, since they represent advance knowledge of the problem, while the constraints relating to the image characteristics (grey levels and gradients) are weaker and subject to more uncertainty. Consequently, given that $\alpha$, $\delta$ = 1, the other two weights were selected as follows: $\beta$, $\gamma$ = 10$^{-2}$.

It should be noted that, generally speaking, the constrains of approximation and regularity P ($a_{mn}$, $b_{mn}$), S ($a_{mn}$, $b_{mn}$) turn out to be sufficient for estimating the epicardial surface $\Sigma$. This may be obtained by minimizing a more general cost functional having the expression:

$$C (a_{mn}, b_{mn}) = P (a_{mn}, b_{mn}) + \lambda S (a_{mn}, b_{mn})$$

where the coefficient $\lambda$ serves to control the degree of regularity and it is preferable $\lambda$ = 1. In view of its greater computational simplicity, the use of the general cost functional allows to decrease the computation time. On the other hand, it does not take into consideration all the information available in images. Consequently, the choice of points $D_i$ and possible errors in the positioning take on greater significance compared with the case where the full cost functional is used. In order to limit these effects, the number of points used may be increased. This, however, is made to the expense of a more complicated interaction with the operator.

The functional can be minimized by various numeric methods, advantageously by means of the Brent method for example, which obviates the calculation of the derivatives of the functional and is suitable for computationally efficient execution.

Subsequently, in step 400, the mathematical surface $\Sigma$ estimated in step 300 is rasterized; in other words, the three-dimensional mathematical representation which can be described by means of vectors in the system of spherical coordinates ($\varphi$,$\theta$) relative to the pole G is converted into a three-dimensional image comprising a discrete number of voxels.

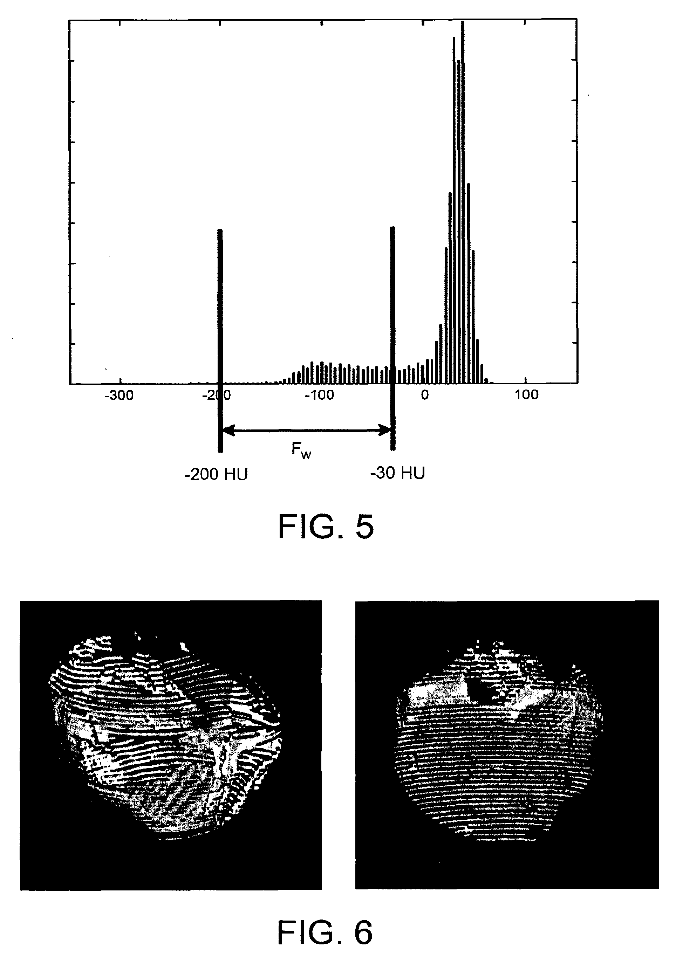

Finally, the calculation of the volume of fat takes place in step 500, and includes the counting of the voxels which are within the estimated discretized pericardial surface $\Sigma_d$ and have a density within a window [$I_{low}$, $I_{high}$] characteristic of fatty tissue, indicated by $F_W$ in Figure 5.

In the case of X-ray tomography, the window $F_w$ is typically in the range from about -200 HU to about -30 HU.

Figure 6 shows two 3D views (front left and rear right) of the fat deposits identified in this way in the whole epicardial region.

Advantageously, it is also possible to provide the value of the ratio $VN_H$, where $V_H$ is the total volume of the heart.

Conveniently, it is also possible to carry out more refined analyses such as the calculation of the regional distribution of fat deposits.

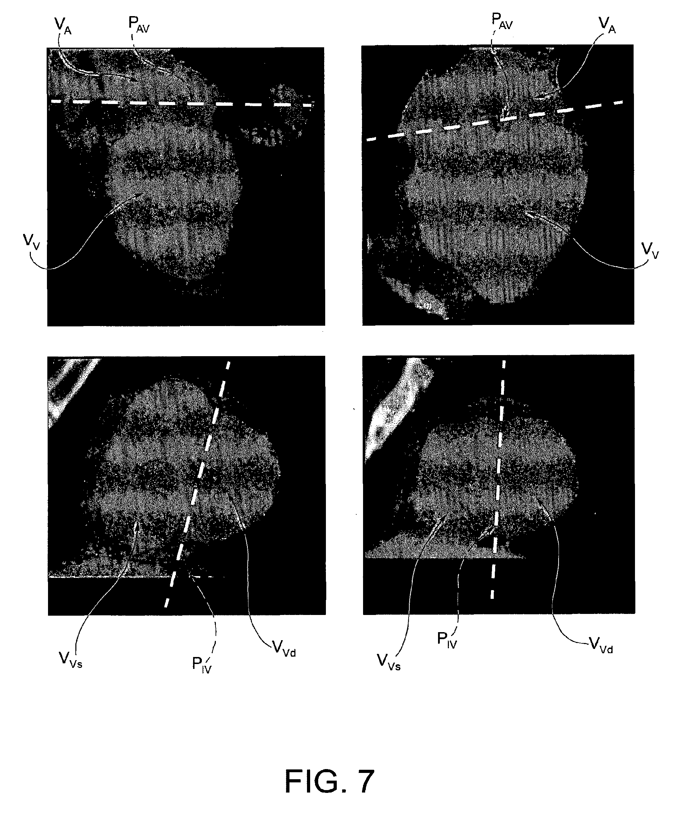

For this purpose, the operator identifies the atrioventricular plane $P_{AV}$ (in two long-axis sections as shown in Figure 7, for example), and the volumes of fat in the ventricular region $V_v$ and in the atrial region $V_A$ are calculated. Similarly, by identifying the interventricular plane $P_{IV}$ (for example in two short-axis views, also shown in Figure 7), the volumes $V_{Vs}$ and $V_{Vd}$ of fat in the left and right ventricular regions respectively are calculated.

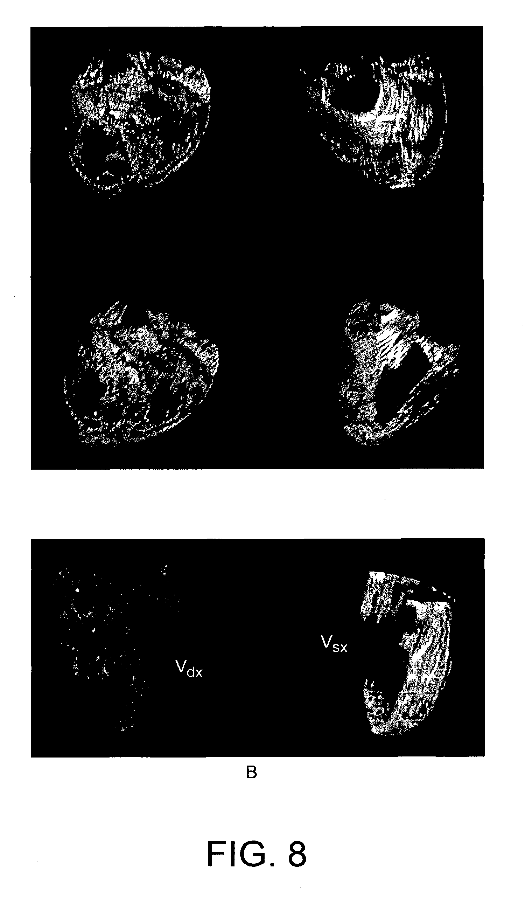

Figure 8 shows some 3D views of the fat deposits in various cardiac regions. In particular, the upper plate shows the ventricular region from different viewpoints, while the right and left ventricular regions are shown in cross section in the lower plate.

To summarize, the method proposed by the invention can be used to determine the volume of cardiac fat with reduced interaction with the operator, and is characterized by high repeatability, low inter- and intra-operator variability, and substantial rapidity of execution.

Furthermore, the method proposed by the invention can be applied to volumetric image data obtained either with or without the use of contrast medium.

Advantageously, the method described can be used to improve the process of diagnosing cardiovascular pathologies. In particular, in the case of X-ray tomography, it does not require any further exposure to ionizing radiation than the standard procedures. The method can also be used by non-medical personnel in clinical practice.

Because of the automation of the process of determining the volume of cardiac fat, the subject of the invention can be used for studying cardiovascular diseases in large populations.

It should be noted that the embodiment of the present invention proposed in the preceding discussion is purely exemplary and does not limit the present invention. A person skilled in the art can easily apply the present invention in different embodiments which do not depart from the principles described above, and which are therefore included in the present patent.

This is particularly true of the possibility of applying the method proposed by the invention to three-dimensional images obtained by different 3D imaging methods.

In particular, no essential modifications are required for magnetic resonance, except in respect of the lower spatial resolution (the method is in itself capable of operating at spatial resolutions other than those of standard CT). It may be necessary to adjust (i) the parameters for the grey window of the fat (in MRI these values depend on the excitation sequence, whereas in CT the variability of the fat window is practically negligible), (ii) the values of the weights $\alpha$, $\beta$, $\gamma$, $\delta$ of the cost functional. It should be borne in mind that some of the constraints expressed in the cost functional can be removed, if necessary, by setting the corresponding coefficients to zero. This may be convenient in cases where the information provided by the grey levels (as in the case of some MRI excitation sequences) and/or by the contours may be confusing. In an extreme case, the method can be used by disregarding the grey level (assuming that $\beta=\gamma=0$) and using only the contours supplied at the input.

Naturally, the principle of the invention remaining the same, the forms of embodiment and details of construction may be varied widely with respect to those described and illustrated, which have been given purely by way of non-limiting example, without thereby departing from the scope of protection of the present invention as defined by the attached claims.

**Claims**

1. Method for determining the volume of epicardial fat of a heart, comprising:

   - acquiring (100) a plurality of volumetric image data (S) representing the heart;
   - morphologically characterizing a volume of interest represented by said volumetric image data, including identifying (200) a predetermined number of reference contours ($P_i$) of the heart;
   - estimating (300) the epicardial surface, $\Sigma$, according to a three-dimensional model based on a series expansion of vectorial spherical harmonic functions from said reference contours ($P_i$);
   - determining (500) the volume of epicardial fat based on the voxels which are located inside the estimated epicardial surface, $\Sigma$, and which have a grey level within a predetermined range, characteristic of fatty tissue, and
   - estimating (300) the epicardial surface includes:
   - expressing the surface as a series of vector spherical harmonic functions in parametric form, as

$$\mathbf{x}(\varphi, \theta) = \sum_{n=0}^{N} \sum_{m=0}^{n} [\mathbf{a}_{mn} \cos m\varphi + \mathbf{b}_{mn} \sin m\varphi] P_{mn}(\cos(\theta))$$

where $\mathbf{a}_{mn}$, $\mathbf{b}_{mn}$ are the vector coefficients of the series, $P_{mn}(t)$ are the associated Legendre polynomials of degree n and order m, the number N is the order of the surface, and $\varphi, \theta$ are the spherical coordinates with respect to the centroid of the reference contours; **characterised in that** it comprises determining the coefficients of the series expansion of vector spherical harmonic functions by minimizing a cost functional

$$\mathcal{C}(a_{mn}, b_{mn}) = \alpha \mathcal{P}(a_{mn}, b_{mn}) + \beta \mathcal{G}(a_{mn}, b_{mn}) + \gamma \mathcal{E}(a_{mn}, b_{mn}) + \delta \mathcal{S}(a_{mn}, b_{mn})$$

wherein

- $\mathcal{P}$ is a functional representing an error of approximation of the epicardial surface ($\Sigma$) by the reference contours ($P_i$);
- $G'$ is a functional representing the presence within the surface of grey levels outside said predetermined range of grey levels of the fatty tissue;
- $\varepsilon$ is a functional representing the gradients of grey levels at the interface of the epicardial surface;
- $S$ is a functional representing the regularity of the surface, and
the coefficients $\alpha$, $\beta$, $\gamma$, $\delta$ are chosen with the values $\alpha$, $\delta = 1$ and $\beta$, $\gamma = 10^{-2}$.

2. Method according to Claim 1, wherein the functional $\mathcal{P}$ is calculated as the sum of the distances $d_i$ of the reference contours ($P_i$) from the epicardial surface, $\Sigma$, according to the expression:

$$\mathcal{P} = \frac{1}{K} \sum_{i} d_i^2 (D_i, \Sigma)$$

3. Method according to Claim 1, wherein the functional $G'$ is calculated according to the expression:

$$\mathcal{G} = \int_{\Omega} H(I_{high} - I(x, y, z))(I_{high} - I(x, y, z))^2 dxdydz +$$
$$+ \int_{\Omega} H(I(x, y, z) - I_{low})(I_{low} - I(x, y, z))^2 dxdydz$$

where H is the unitary step function, I(x,y,z) is the grey level function, $I_{low}$ is the lower bound of the predetermined range of grey levels, and $I_{high}$ is the upper bound of the preset range of grey levels.

4. Method according to Claim 1, wherein the functional $\varepsilon$ is calculated according to the expression:

$$\mathcal{E} = \int_{\Sigma} \frac{1}{1 + |\nabla I(x, y, z)|^2} |\nabla I(x, y, z)| d\Sigma$$

where I(x,y,z) is the grey level function.

5. Method according to Claim 1, wherein the functional $S$ is calculated according to the expression:

$$\mathcal{S} \;=\; \sum_{n}\sum_{m} w_{nm}(a_{mn}^2 + b_{mn}^2)$$

where $w_{mn} = k\,\exp(n/N)$.

6. Method according to any one of the preceding claims, wherein said volumetric image data (S) comprise a collection of two-dimensional image data obtained by tomographic or magnetic resonance scanning, representing a plurality of successive plane views of the heart, which, taken in combination, define a grey level function I(x,y,z).

7. Method according to any one of the preceding claims, wherein morphologically characterizing the volume of interest includes identifying at least three two-dimensional contours (P1-P4) of the heart, comprising the profile of the heart in at least a pair of sectional images taken along a long axis, representing four chambers and two chambers respectively (Asx; Adx; Vsx; Vdx), and in at least one sectional image taken along a short axis at the base of the ventricle (Vdx, Vsx).

8. Method according to Claim 7, wherein said contours (P1-P4) are identified by applying a method of automatic segmentation of the cardiac volume.

9. Method according to Claim 7, wherein said contours (P1-P4) are identified by the marking of a discrete number of boundary points (Di) by an operator and the plotting of an interpolation curve based on the marked points.

10. Method according to any of the preceding claims, wherein determining the volume of epicardial fat includes counting the voxels inside the estimated surface which has been converted into a three-dimensional image data element comprising a discrete number of voxels.

11. Method according to any of the preceding claims, comprising determining the regional distribution of the volume of epicardial fat.

12. System (10) for determining the volume of epicardial fat of a heart, comprising processing means (12) arranged to execute the method according to any of claims 1 to 11.

13. Computer program or group of programs executable by a processing system (10) and comprising one or more code modules for executing the method for determining the volume of epicardial fat according to any of Claims 1 to 11.

14. Computer program product having stored a computer program or group of programs according to Claim 13.

**Patentansprüche**

1. Verfahren zum Bestimmen des Volumens von epikardialem Fett eines Herzens, umfassend:

- Erfassen (100) von mehreren volumetrischen Bilddaten (S), die das Herz darstellen;
- morphologisches Charakterisieren eines interessierenden Volumens, das durch die volumetrischen Bilddaten dargestellt ist, umfassend das Identifizieren (200) einer festgelegten Anzahl von Referenzkonturen (P$_i$) des Herzens;
- Abschätzen (300) der epikardialen Fläche, $\Sigma$, nach einem dreidimensionalen Modell, das auf einer Reihenentwicklung von vektoriellen sphärischen harmonischen Funktionen der Referenzkonturen (P$_i$) beruht;
- Bestimmen (500) des Volumens von epikardialem Fett, das auf den Voxeln, die sich innerhalb der abgeschätzten epikardialen Fläche befinden, beruht, $\Sigma$, und die eine Graustufe in einem festgelegten Bereich aufweisen, der für Fettgewebe charakteristisch ist , und
- Abschätzen (300) der epikardialen Fläche beinhaltet:
- Darstellen der Fläche als eine Reihe von vektoriellen sphärischen harmonischen Funktionen in parametrischer Form, als

$$\mathbf{x}(\varphi,\theta) = \sum_{n=0}^{N} \sum_{m=0}^{n} [\mathbf{a}_{mn} \cos m\varphi + \mathbf{b}_{mn} \sin m\varphi] P_{mn}(\cos(\theta))$$

wobei, $a_{mn}$, $b_{mn}$ die Vektorkoeffizienten der Reihe sind, $P_{mn}$ (t) die zugeordneten Legendre-Polynome von Grad n und Ordnung m sind, die Anzahl N die Ordnung der Fläche angibt, und $\varphi$, $\theta$ die Kugelkoordinaten mit Bezug auf den Schwerpunkt der Referenzkonturen sind;
**dadurch gekennzeichnet, dass** es das Bestimmen der Koeffizienten der Reihenentwicklung der vektoriellen sphärischen harmonischen Funktionen durch das Minimieren eines Kostenfunktionals umfasst

$$\mathcal{C}(a_{mn}, b_{mn}) = \alpha\mathcal{P}(a_{mn}, b_{mn}) + \beta\mathcal{G}(a_{mn}, b_{mn}) + \gamma\mathcal{E}(a_{mn}, b_{mn}) + \delta\mathcal{S}(a_{mn}, b_{mn})$$

wobei

- $\mathcal{P}$ ein Funktional ist, das einen Näherungsfehler der epikardialen Fläche ($\Sigma$) durch die Referenzkonturen ($P_i$) darstellt;
- $G'$ ein Funktional ist, das das Vorhandensein innerhalb der Fläche von Graustufen außerhalb des festgelegten Bereichs von Graustufen des Fettgewebes repräsentiert;
- $\varepsilon$ ein Funktional ist, das die Gradienten der Graustufen an der Grenzfläche der epikardialen Fläche repräsentiert;
- S ein Funktional ist, das die Regelmäßigkeit der Fläche repräsentiert, und
- die Koeffizienten $\alpha$, $\beta$, $\gamma$, $\delta$ mit den Werten $\alpha$, $\delta$ = 1 und $\beta$, $\gamma$ = $10^{-2}$ ausgewählt sind.

2. Verfahren nach Anspruch 1, wobei das Funktional 'P als die Summe der Distanzen $d_i$ der Referenzkonturen ($P_i$) von der epikardialen Fläche, $\Sigma$, nach folgendem Ausdruck berechnet wird:

$$\mathcal{P} = \frac{1}{K} \sum_i d_i^2(D_i, \Sigma)$$

3. Verfahren nach Anspruch 1, wobei das Funktional $G'$ nach folgendem Ausdruck berechnet wird:

$$\mathcal{G} = \int_\Omega H(I_{high} - I(x,y,z))(I_{high} - I(x,y,z))^2 dxdydz +$$
$$+ \int_\Omega H(I(x,y,z) - I_{low})(I_{low} - I(x,y,z))^2 dxdydz$$

wobei H die einheitliche Stufenfunktion ist, I(x,y,z) die Graustufen-Funktion ist, $I_{low}$ die untere Grenze des festgelegten Bereichs von Graustufen ist, und $I_{high}$ die obere Grenze des voreingestellten Bereichs von Graustufen ist.

4. Verfahren nach Anspruch 1, wobei das Funktional $\varepsilon$ nach folgendem Ausdruck berechnet wird:

$$\mathcal{E} = \int_\Sigma \frac{1}{1 + |\nabla I(x,y,z)|^2} |\nabla I(x,y,z)| d\Sigma$$

wobei I(x, y, z) die Graustufenfunktion ist.

**5.** Verfahren nach Anspruch 1, wobei das Funktional S nach folgendem Ausdruck berechnet wird:

$$\mathcal{S} \;=\; \sum_{n}\sum_{m} w_{nm}(a_{mn}^2 + b_{mn}^2)$$

wobei $w_{mn} = k \exp(n/N)$.

**6.** Verfahren nach einem der vorhergehenden Ansprüche, wobei die volumetrischen Bilddaten (S) eine Erfassung von zweidimensionalen Bilddaten umfassen, die durch tomographische oder magnetische Resonanzabtastung gewonnen wurden, wobei sie mehrere Folgeansichten der Ebene des Herzens wiedergeben, die kombiniert eine Graustufenfunktion I(x, y, z) definieren.

**7.** Verfahren nach einem der vorhergehenden Ansprüche, wobei morphologisches Charakterisieren des interessierenden Volumens das Identifizieren von mindestens drei zweidimensionalen Konturen (P1-P4) des Herzens beinhaltet, das das Profil des Herzens in mindestens einem Paar von Schnittbildern, die entlang einer langen Achse aufgenommen wurden, umfasst, das vier Kammern und zwei entsprechende Kammern (Asx; Adx; Vsx; Vdx) und in mindestens einem Schnittbild, das entlang der kurzen Achse am unteren Teil des Ventrikels (Vdx, Vsx)aufgenommen wurde, darstellt.

**8.** Verfahren nach Anspruch 7, wobei die Konturen (P1-P4) durch das Applizieren eines Verfahrens von automatischer Segmentierung des Herzvolumens identifiziert werden.

**9.** Verfahren nach Anspruch 7, wobei die Konturen (P1-P4) durch das Markieren von einer diskreten Anzahl von Randpunkten (Di) durch einen Bediener und das Zeichnen einer Interpolationskurve, die auf den markierten Punkten beruht, identifiziert werden.

**10.** Verfahren nach einem der vorhergehenden Ansprüche, wobei das Bestimmen des Volumens von epikardialem Fett das Zählen der Voxel innerhalb der geschätzten Fläche, die in ein dreidimensionales Bilddatenelement umgewandelt wurde, welches eine diskrete Anzahl von Voxeln umfasst, beinhaltet.

**11.** Verfahren nach einem der vorhergehenden Ansprüche, das das Bestimmen der örtlichen Verteilung des Volumens von epikardialem Fett umfasst.

**12.** System (10) zum Bestimmen des Volumens von epikardialem Fett eines Herzens, das Verarbeitungsmittel (12) umfasst, die angeordnet sind, um das Verfahren nach einem der Ansprüche 1 bis 11 durchzuführen.

**13.** Computerprogramm oder Gruppe von Programmen, die durch ein Verarbeitungssystem (10) ausführbar sind und einen oder mehr Codebausteine zum Ausführen des Verfahrens zum Bestimmen des Volumens von epikardialem Fett nach einem der Ansprüche 1 bis 11 umfassen.

**14.** Computerprogrammprodukt, das ein Computerprogramm oder eine Gruppe von Programmen nach Anspruch 13 gespeichert hat.


**Revendications**

**1.** Procédé pour déterminer le volume de tissu adipeux épicardique d'un coeur, comportant

- l'acquisition (100) d'une pluralité de données d'images volumétriques (S) représentant le coeur ;
- la caractérisation morphologique d'un volume concerné représenté par lesdites données d'images volumétriques, comprenant l'identification (200) d'un nombre prédéterminé de contours de référence ($P_i$) du coeur ;
- l'estimation (300) de la surface épicardique, $\Sigma$, suivant un modèle tridimensionnel reposant sur un développement en série de fonctions harmoniques sphériques vectorielles, d'après lesdits contours de référence ($P_i$) ;
- la détermination (500) du volume de tissu adipeux épicardique d'après les voxels qui sont situés à l'intérieur de la surface épicardique estimée, $\Sigma$, et qui ont un intervalle de niveaux de gris prédéterminé, caractéristique de tissus adipeux, et

- l'estimation (300) de la surface épicardique comprenant :
- l'expression de la surface par une série de fonctions harmoniques sphériques vectorielles sous une forme paramétrique, à savoir

$$X(\varphi, \theta) = \sum_{n=0}^{N} \sum_{m=0}^{n} [a_{mn} \cos m\varphi + b_{mn} \sin m\varphi] P_{mn}(\cos(\theta))$$

où $a_{mn}$, $b_{mn}$ sont les coefficients vectoriels de la série, $P_{mn}(t)$ sont les polynômes de Legendre associés, de degré n et d'ordre m, le nombre N est l'ordre de la surface, et $\varphi$, $\theta$ sont les coordonnées sphériques par rapport au centroïde des contours de référence ;
**caractérisé en ce qu'**il comporte une détermination des coefficients du développement en série de fonctions harmoniques sphériques vectorielles en minimisant une fonctionnelle coût

$$C(a_{mn}, b_{mn}) = \alpha P(a_{mn}, b_{mn}) + \beta G(a_{mn}, b_{mn}) + \gamma \mathcal{E}(a_{mn}, b_{mn}) + \delta S(a_{mn}, b_{mn})$$

où
- *P* est une fonctionnelle représentant une erreur d'approximation de la surface épicardique ($\Sigma$) par les contours de référence ($P_i$) ;
- *G* est une fonctionnelle représentant la présence, sur la surface, de niveaux de gris hors dudit intervalle de niveaux de gris des tissus adipeux ;
- $\varepsilon$ est une fonctionnelle représentant les gradients de niveaux de gris à l'interface de la surface épicardique ;
- *S* est une fonctionnelle représentant la régularité de la surface, et
les coefficients $\alpha$, $\beta$, $\gamma$, $\delta$ sont choisis avec les valeurs $\alpha$, $\delta = 1$ et $\beta$, $\gamma = 10^{-2}$.

2. Procédé selon la revendication 1, dans lequel la fonctionnelle *P* est calculée comme somme des distances $d_i$ des contours de référence ($P_i$) par rapport à la surface épicardique, $\Sigma$, suivant l'expression :

$$P = \frac{1}{K} \sum_i d_i^2(D_i, \Sigma)$$

3. Procédé selon la revendication 1, dans lequel la fonctionnelle *G* est calculée suivant l'expression :

$$G = \int_{\Omega} H(I_{high} - I(x,y,z)(I_{high} - I(x,y,z))^2 dydydz +$$

$$+ \int_{\Omega} H(I(x,y,z - I_{low})(I_{low} - I(x,y,z))^2 dydydz$$

où H est la fonction échelon-unité, I(x,y,z) est la fonction de niveau de gris, $I_{low}$ est la limite inférieure de l'intervalle de niveau de gris prédéterminé, et $I_{high}$ est la limite supérieure de l'intervalle de niveaux de gris prédéterminé.

4. Procédé selon la revendication 1, dans lequel la fonctionnelle $\varepsilon$ est calculée suivant l'expression :

$$\mathcal{E} = \int_{\Sigma} \frac{1}{1 + |\nabla I(x,y,z)|^2} |\nabla I(x, y, z)| d\Sigma$$

où I(x,y,z) est la fonction de niveau de gris

5. Procédé selon la revendication 1, où la fonctionnelle *S* est calculée suivant l'expression :

$$S = \sum_n \sum_m w_{\mathrm{nm}}(a_{mn}^2 + b_{mn}^2)$$

où $w_{mn} = k \exp(n/N)$.

6. Procédé selon l'une quelconque des revendications précédentes, dans lequel lesdites données d'images volumétriques (S) comprennent un ensemble de données d'images bidimensionnelles obtenues par tomographie ou par résonance magnétique, représentant une pluralité de vues en plan successives du coeur, lesquelles, après avoir été combinées, définissent une fonction de niveau de gris I(x,y,z).

7. Procédé selon l'une quelconque des revendications précédentes, dans lequel la caractérisation morphologique du volume concerné comprend une identification d'au moins trois contours bidimensionnels (P1-P4) du coeur, dont le profil du coeur sur au moins une paire d'images en coupe prises sur un grand axe, représentant respectivement quatre chambres et deux chambres (Asx ; Adx ; Vsx ; Vdx), et sur au moins une image en coupe prise sur un petit axe à la base du ventricule (Vdx, Vsx).

8. Procédé selon la revendication 7, dans lequel lesdits contours (P1-P4) sont identifiés en appliquant un procédé de segmentation automatique du volume cardiaque.

9. Procédé selon la revendication 7, dans lequel lesdits contours (P1-P4) sont identifiés par la réalisation d'un nombre discret de points aux limites (Di) par un opérateur et par le tracé d'une courbe d'interpolation sur la base des points marqués.

10. Procédé selon l'une quelconque des revendications précédentes, dans lequel la détermination du volume de tissu adipeux épicardique comprend un comptage des voxels à l'intérieur de la surface estimée qui a été convertie en élément de données d'image tridimensionnelle comprenant un nombre discret de voxels.

11. Procédé selon l'une quelconque des revendications précédentes, comportant une détermination de la répartition régionale du volume de tissu adipeux épicardique.

12. Système (10) pour déterminer le volume de tissu adipeux épicardique d'un coeur, comportant un moyen de traitement (12) conçu pour exécuter le procédé selon l'une quelconque des revendications 1 à 11.

13. Programme ou groupe de programmes informatique(s) exécutable(s) par un système de traitement (10) et comportant un ou plusieurs modules de codage pour exécuter le procédé afin de déterminer le volume de tissus adipeux épicardiques selon l'une quelconque des revendications 1 à 11.

14. Programme informatique dans lequel est stocké un programme informatique ou un groupe de programmes selon la revendication 13.

# FIG. 1

START

2D IMAGES ACQUISITION — 100

CONTOURS IDENTIFICATION — 200

SURFACE ESTIMATION — 300

RASTERIZATION — 400

VOLUME DETERMINATION — 500

END

FIG. 2

FIG. 3

FIG. 4

$F_w$

-200 HU          -30 HU

FIG. 5

FIG. 6

FIG. 7

B

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BANDEKAR, A.N. ; NAGHAVI, M. ; KAKADIARIS, I.A.** Automated Pericardial Fat Quantification in CT Data. *Engineering in Medicine and Biology Society, 2006. EMBS '06. 28th Annual International Conference of the IEEE,* 2006, 932-935
- **DAMINI DEY ; YASUYUKI SUZUKI ; SHOJI SUZUKI ; MUNEO OHBA ; PIOTR J. SLOMKA ; DONNA POLK ; LESLEE J. SHAW ; DANIEL S. BERMAN.** Automated Quantization of Pericardiac Fat From Noncontrast CT. *Investigative Radiology,* February 2008, vol. 43 (2
- **COPPINI G ; FAVILLA R ; LAMI E ; MARRACCINI P ; MORONI D ; SALVETTI O.** Regional epicardial fat measurement: Computational methods for cardiac CT imaging. *Transactions on Mass-Data Analysis of Images and Signals,* 2009, vol. 1, 101-110
- **COPPINI G ; FAVILLA R ; MARRACCINI P. ; MORONI D. ; PIERI G.** Quantification of Epicardial Fat by Cardiac CT Imaging. *The Open Medical Informatics Journal,* 2010, vol. 4, 126-135
- **G. COPPINI.** P2505 - Computer technology for the quantification of pericardial fat assessed though cardiac CT. *ESC Congress,* 2010
- **G. COPPINI et al.** Computational methods for pericardial fat evaluation. *MDA 2011 - Advances in Mass data Analyses of Images and Signals in Medicine, Biotechnology, Chemistry and food Industry, 6th Internatinal Conference,* 01 January 2011, 38-49